# EUROPEAN PATENT APPLICATION

(11) **EP 4 574 022 A1**
(43) Date of publication of application: **25.06.2025**
(21) Application number: 23307294.1
(22) Date of filing: 20.12.2023
(51) Int. Cl.: A61B 5/00, G06T 7/00, A61B 5/0295

(54) **MONITORING ONE OR MORE INDICATORS OF A PART OF THE BODY**

(71) Applicant: Université de Bourgogne, 21078 Dijon (FR)
(72) Inventor: BENEZETH, Yannick, Dijon (FR); GOURILLON, Matthieu, Saint-Loup-Géanges (FR); DUBOIS, Julien, Quétigny (FR)
(74) Representative: Bandpay & Greuter

(57) **Abstract**

The disclosure notably relates to computer-implemented method for monitoring one or more indicators of a part of the body from images representing the skin of the part of the body. The method comprises obtaining a sequence of images representing the skin of the part of the body. The method also comprises estimating a reference remote photoplethysmography (rPPG) signal of the skin from groups of pixels of the images. The method also comprises obtaining a temporal rPPG signal for each pixel of a respective group of pixels by obtaining a linear combination of pixel characteristics of the group of pixels. The method also comprises filtering each temporal rPPG signal of the respective group of pixel based on the reference rPPG signal, thereby obtaining respective rPPG signals for each group of pixels.

## Description

### TECHNICAL FIELD

The disclosure relates to the field of computer programs and systems, and more specifically to a method, system and program for monitoring one or more indicators of a part of the body from images representing the skin of the part of the body.

### BACKGROUND

A number of systems and programs exist for monitoring the skin of a part of the body, for example an extremity such as feet in a variety of contexts, such as medical, pharmaceutical, or other contexts such as artificial vision or industrial systems. Traditional monitoring methods are often based on subjective visual observations, which may yield false positives or incorrect measurements. Moreover, traditional methods may perform manual measurements which are inaccurate, but also increase the risk of infection and/or pain to a user due to the need of contact with the skin, for instance in the case where the skin presents a wound such as an ulcer.

Within this context, there is still a need for an improved method for monitoring one or more indicators of a part of the body from images representing the skin of the part of the body.

### SUMMARY

It is therefore provided a computer-implemented method for monitoring one or more indicators of a part of the body from images representing the skin of the part of the body. The method comprises obtaining a sequence of images representing the skin of the part of the body. The method also comprises estimating a reference remote photoplethysmography (rPPG) signal of the skin from groups of pixels of the one or more images. The method also comprises obtaining a temporal rPPG signal for each respective group of pixels by obtaining a linear combination of pixel characteristics of the group of pixels. The method also comprises filtering each temporal rPPG signal on the respective group of pixels based on the reference rPPG signal, thereby obtaining respective rPPG signals for each group of pixels. The respective rPPG signals are suitable to be used for obtaining one or more indicators of a part of the body from images representing the skin based on the obtained respective rPPG signals.

The method may comprise one or more of the following:
- filtering each temporal rPPG signal comprises applying a lock-in amplifier on the group of pixels;
- the method further comprises, prior to obtaining the temporal rPPG signal, applying a filter on the groups of pixels, such as a low-pass filter or a bilateral filter;
- the estimation of the reference rPPG signal comprises determining a region of interest enclosing the skin, and measuring a pixel mean n pixels of the region of interest;
- the method further comprises determining one or more segments of the image representing the skin from the region of interest;
- the sequence of images may comprise RGB images, the pixel characteristics corresponding to RGB values of a respective RGB image and/or the sequence of images may comprise monochrome images, the pixel characteristics corresponding to luminosity levels represented on a respective monochrome image;
- the sequence of images comprises RGB images, and obtaining the linear combination of pixel characteristics comprises maximizing a correlation between the RGB color values and the reference rPPG signal;
- the method further comprises, prior to estimating the reference PPG signal, decreasing the resolution of the images to a first resolution, the first resolution being lower than the dimension of the images, obtaining the reference rPPG signal, obtaining and filtering each the temporal rPPG signal, the method further increasing the resolution of the images from the first resolution to a second resolution, the second resolution being larger than the first resolution, if the respective rPPG signal satisfies a predetermined threshold.

It is further provided a method of use of the method. The method of use comprises acquiring, by an image sensor, a sequence of images representing the skin of the part of the body. The method of use also comprises using the method of any one of claims 1 to 6 for obtaining one or more indicators of a part of the body from images representing the skin based on the obtained respective rPPG signals.

The method of use may comprise one or more of the following:
- the one or more indicators comprise one or more of a perfusion index or a magnitude;
- the method of use may further comprise monitoring a wound on the skin based on the obtained respective rPPG signal.

It is further provided a computer program comprising instructions which, when the program is executed by a computer, cause the computer to carry out the method or the method of use.

It is further provided a computer readable storage medium having recorded thereon a computer program.

It is further provided a system comprising a processor coupled to a memory, the memory having recorded thereon the computer program.

### BRIEF DESCRIPTION OF THE DRAWINGS

Non-limiting examples will now be described in reference to the accompanying drawings, where:
- FIG. 1 shows a flowchart of an example of the method;
- FIG. 2 shows a flowchart of an example of the method of use;
- FIG. 3 shows an example of the system; and
- FIG.s 4 and 5 shows examples of the method.

### DETAILED DESCRIPTION

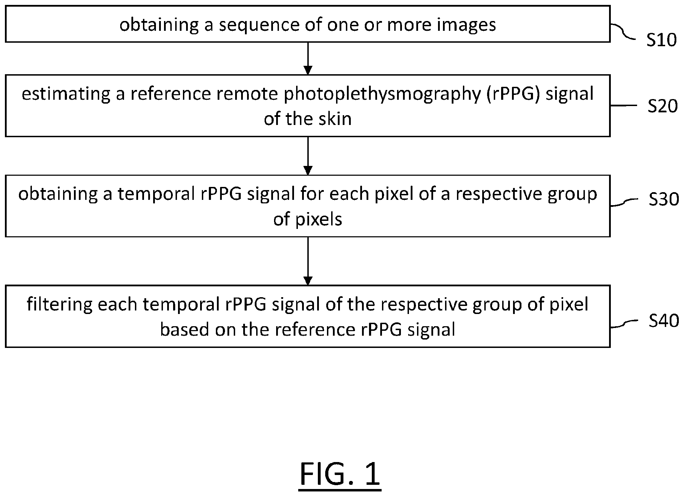

With reference to the flowchart of FIG. 1, it is proposed a computer-implemented method for monitoring one or more indicators of a part of the body from images representing the skin of the part of the body. The method comprises obtaining S10 a sequence of one or more images. The images represent the skin of the part of the body. The method also comprises estimating S20 a reference remote photoplethysmography (rPPG) signal of the skin from groups of pixels of the images. The method also comprises obtaining S30 a temporal rPPG signal for each respective group of pixels by obtaining a linear combination of pixel characteristics of the group of pixels. The method also comprises filtering S40 each temporal rPPG signal of the respective group of pixels based on the reference rPPG signal, thereby obtaining respective rPPG signals for each group of pixels.

Such a method improves monitoring one or more indicators of a part of the body from images representing the skin of the part of the body.

The use of the respective rPPG signal for monitoring a part of the body offers several advantages. As the respective rPPG signal is obtained from the images (captured for example through simple RGB or grayscale image cameras), no complicated setup is required to obtain the signal and is therefore inexpensive and requires no specific training for a user to acquire the data. The respective rPPG signal is correlated to dynamical changes in blood volume under the skin of the part of the body, and thus is strongly correlated to several indicators on the skin, for example the health of the skin. The indicators thus enable objective, reproducible assessment of changes of the monitored part of the body, without being painful for the patient as it is noninvasive and remote from the body skin. The method uses a particularly accurate way of obtaining the rPPG signal, all thanks to the specific steps performed by the method. The method improves the obtention of rPPG signals for each pixel, which is specially challenging because the level of the signal of interest is significantly lower than the level of acquisition noise and quantification noise. This problem is usually solved by calculating a spatial average of the signals in the region of interest, but this step cannot be applied in our case as it would reduce the spatial resolution of our indicator map. The method applies the temporal filtering to each rPPG signal based on the reference rPPG signal. This results in that the method significantly increases the quality of the temporal rPPG signals for each pixel while maintaining the spatial resolution of the original images. The temporal rPPG signals allows to concentrate on the pixel characteristics of each pixel of the group compared to the reference rPPG signal. As the temporal rPPG signal is filtered based on the reference rPPG signal, the method takes into account the pixel characteristics of the groups of pixels without averaging noise, thereby achieving a highly accurate respective rPPG signal.

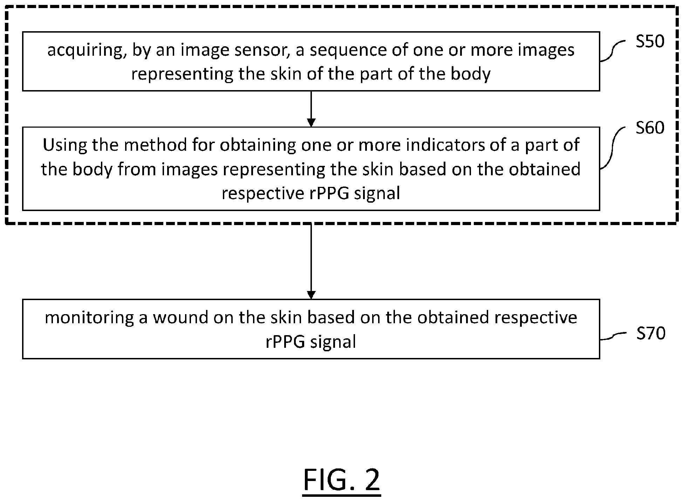

With reference to FIG. 2, it is further provided a method of use of the method. The method of use comprises acquiring S50, by an image sensor, a sequence of images representing the skin of the part of the body. The image sensor may be an RGB sensor or a luminosity sensor, in which case the images may be color images or grayscale images. The image sensor may be positioned so as to capture at least a portion of the part of the body. The method of use also comprises using S60 the method for obtaining one or more indicators of a part of the body from images representing the skin based on the obtained respective rPPG signal.

The one or more indicators may comprise one or more of a perfusion index, a magnitude of the temporal rPPG signals or a correlation map between the reference rPPG signal and the respective rPPG signals. The perfusion index is a ratio between a pulsating component over a non-pulsating component of the respective rPPG signals. The magnitude of the temporal rPPG signals may be computed from a mean or median of the separation between the crest and the trough of each respective rPPG signal after normalization (for example by dividing a respective rPPG signal over an average value). The correlation map provides an indicator that describes the quality of the respective rPPG signals, and hence provides an indicator of vascularization.

The method of use thus allows to obtain quantitative measurements on the skin. Contrary to traditional manual methods , which perform at least some of those measurements manually (for example by using a ruler near the wound or an applicator moistened cotton for depth)and which are imprecise, painful and uncomfortable for the patient, the method of use provides an objective indicator of the skin of the part of the body thanks to the one or more indicators, which are more complete and informative than a manual assessment with a ruler. Additionally, contact with tools such as rulers or ribbons increases the risk of contamination by bacterial propagation. In addition the information collected by the traditional methods are limited, subjective and subject to human error. In contrast, the method of use requires no contact with the patient. Indeed, the method of use only requires to use an image sensor, for example from a camera such as a web cam camera or a camera installed on a phone.

The method of use may further comprise monitoring S70 a wound on the skin based on the obtained respective rPPG signal, for example a chronic wound such as an ulcer on a foot. The method of use may display the one or more indicators obtained based on the respective rPPG signals, for example as an image showing the obtained one or more indicators (for example, the perfusion index) overlayed over positions of the skin of the part of the body. Thus, the method of use improves the monitoring and diagnosis of a wound. Indeed, the treatment of chronic wounds is an important health issue with an increasing demand for performing efficient monitoring of wounds such as foot ulcers. Consequently, optimal treatment can be performed based on the measurements obtained by the method of use to reduce the mortality rate. In Europe alone, around six million people with diabetes suffer from chronic wounds, including foot ulcers, which can lead to amputation of a limb and result in a mortality rate of 30% during the first year, similar to that of certain cancers. The method of use moreover improves over traditional monitoring methods, which are often based on subjective visual observations and manual measurements which are inaccurate and can also be painful for patients.

The methods of the present disclosure are computer-implemented. This means that steps (or substantially all the steps) of the methods disclosed herein are executed by at least one computer, or any system alike. Thus, steps of any of the methods are performed by the computer, possibly fully automatically, or, semiautomatically. In examples, the triggering of at least some of the steps of the methods may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method of the present disclosure is to perform the method with a system adapted for this purpose. The system comprises a processor coupled to a memory and may comprise a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

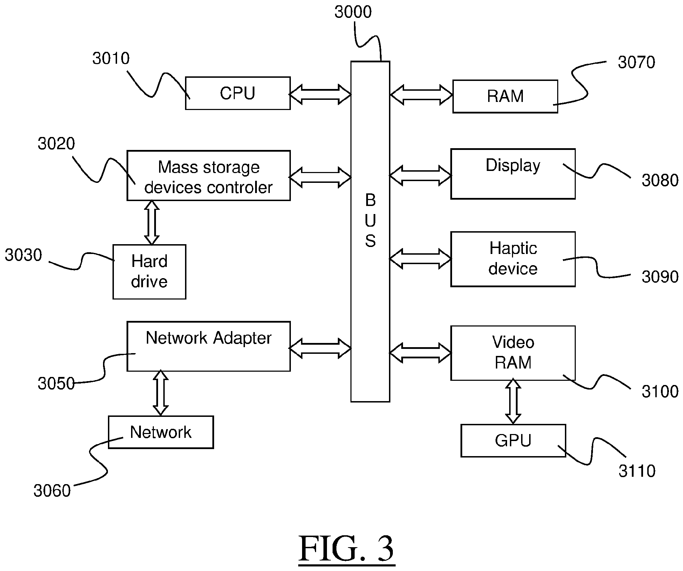

FIG. 3 shows an example of the system, wherein the system is a client computer system, *e.g.* a workstation of a user.

The client computer of the example comprises a central processing unit (CPU) 3010 connected to an internal communication BUS 3000, a random access memory (RAM) 3070 also connected to the BUS. The client computer is further provided with a graphical processing unit (GPU) 3110 which is associated with a video random access memory 3100 connected to the BUS. Video RAM 3100 is also known in the art as frame buffer. A mass storage device controller 3020 manages accesses to a mass memory device, such as hard drive 3030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 3050 manages accesses to a network 3060. The client computer may also include a haptic device 3090 such as cursor control device, a keyboard or the like. A cursor control device is used in the client computer to permit the user to selectively position a cursor at any desired location on display 3080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the methods of the present disclosure. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the methods by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the methods to be performed on the cloud computing environment.

By "sequence of images" it is meant that the images are arranged in an order, for example in a chronological order, or according to a given index. Obtaining the sequence may comprise acquiring the sequence from a sensor, for example in real time; the images of the sequence are ordered according to the time in which a respective image is acquired. Alternatively, obtaining the sequence may comprise retrieving the images from non-volatile memory, or from a distant computer such as a server.

Each image of the sequence represents the skin of the part of the human body. In other words, each image comprises groups of pixels representative of the skin of the part of the human body, e.g., a healthy skin and/or part of the skin presenting a wound such as an ulcer. The part of the human body may be any part, for example the foot, a portion of the arm, the hand, face, chest.

In the context of the present disclosure, remote photoplethysmography (rPPG) refers to a non-invasive optical technique to detect blood-volume variations in the microvascular bed of the skin with images obtaining from cameras, by detecting variations on the light absorbed by the skin. An increase in blood-volume decreases the intensity of the light reflected from the skin, as blood has a higher absorption than bloodless skin.

The reference remote rPPG signal of the skin S20 is estimated from groups of pixels of the images. That is, the reference remote rPPG signal represents the reflected light from the skin as represented on the group of pixels. By "group of pixels" it is meant a collection of at least one pixel of a respective image of the sequence, for example at least one image of the sequence or all of the images of the sequence. The group of pixels may be of any shape, for example, it may be a rectangular collections of pixels, for example a rectangle of 15x15 pixels or more. Each group of pixels represents the skin of the part of the body. In other words, at least one pixel comprises a color indicative of the presence of the skin of the part of the body. The group of pixels may comprise neighboring pixels. In other words, two neighboring pixels may be located on consecutive 2D coordinates of a respective image of the images.

The temporal rPPG signal S30 is obtained, for each pixel of the respective group of pixels, by obtaining a linear combination of pixel characteristics of the groups of pixels. In other words, the method obtains, for each pixel of the group, the temporal rPPG signal is a result of the linear combination. By "pixel characteristic" it is meant a property belonging to each individual pixel of the group of pixels. For example, the pixel characteristic may include the luminosity of a part of the body represented on each pixel of the group. The luminosity may be represented on a monochromatic scale such as grayscale. Additionally or alternatively, the pixel characteristic may include a grayscale level of each pixel or a color level of the pixel, for example, a color level. For example, the images may comprise RGB images. In such a case, each pixel may comprise a triplet of pixel characteristics, each pixel characteristic of the triplet being a respective RGB color value, that is, one of a red, green or blue color value.

The sequence may comprise RGB images. In such a case the pixel characteristics may correspond to RGB values of each respective color channel of a respective RBG image, in other words, a value for the red color channel, a value for the blue color channel and a value for the blue color channel of the RGB image. Alternatively, the sequence may comprise monochrome images. The monochrome image may be a grayscale image. The pixel characteristics may correspond to luminosity levels represented on a respective monochrome image. For example, the monochrome image may be a grayscale image and the luminosity levels may be represented according to grayscales, for example the lowest luminosity may be represented with 0 and the highest luminosity as 255, while intermediate luminosity levels may be represented as integer values in the range between [0, 255].

By linear combination, it is meant that the method may sum the pixel characteristics of the groups of pixels by multiplying, for each pixel, each pixel characteristic by a weight or constant and adding the results. The linear combination may be obtained in any manner, that is, the weight or constant for a respective pixel characteristic may be determined in any manner. For example, the weight or constant for a respective pixel may be determined according to an optimization procedure. In examples, the sequence may comprise RGB images; the linear combination may comprise performing a weighted sum of RGB values of each respective color channel of a respective RGB image.

The method filters each temporal rPPG signal S40 of the respective group of pixels based on the reference rPPG signal. The method may apply a temporal filter (as known in the field of image processing and signal processing) on the respective rPPG signals The method thereby obtains respective rPPG signals for each group of pixels. In other words, for pixel of the group, a respective rPPG signal is the result of applying the filter on a corresponding temporal rPPG signal.

The method thus improves the monitoring of one or more indicators of the part of the body. Indeed, as the method obtains a sequence of images representing the skin of the part of the body, the method is noninvasive as there is no contact required with the body.

Moreover, the temporal rPPG signal for each pixel is filtered based on the reference rPPG signal, the method is highly accurate for obtaining relevant data that gives information on the health of the skin, for example when a wound is present on the skin. The one or more indicators estimated from the respective rPPG signals may be presented in the form of a 2D map, e.g., the 2D map may comprise a value of a respective rPPG signal at a position of the 2D map corresponding to the position of a pixel on the sequence of images corresponding to the respective rPPG signal.

The sequence of images may be spaced by any kind of time difference. For example, sequence may comprise subsequences of images separated by a space of days or weeks. The method may obtain 2D maps for each respective subsequence. Thus, a practician may have a basis for performing the comparison of the evolution of the health of the skin on the part of the body according to the in which each respective subsequence is acquired.

The sequence of images may comprise RGB images. Obtaining the linear combination of pixel characteristics may comprise maximizing a correlation between the RGB color values and the reference rPPG signal. The method may perform the maximization of the correlation according to a semi-blind source separation. The method may introduce the reference rPPG signal to guide the maximization of the correlation. The maximization of the correlation may be performed by any optimization method, for example as described in the publication *"*Heart rate estimation using remote photoplethysmography with multi-objective optimization"; Richard Macwan, Yannick Benezeth, Alamin Mansour; Biomedical Signal Processing and Control; 2019*.* This results on a highly accurate determination of the reference rPPG signal which is non invasive and that can be executed on images coming from standard cameras, as the method only requires RGB color information.

Filtering each temporal rPPG signal S40 may comprise applying a lock-in amplifier on the group of pixels. As known in the field of signal processing, the lock-in amplifier is a temporal filter configured to extract a signal based on a known carrier. In the case of the method, the lock-in amplifier extracts the temporal rPPG signal from the group of pixels based on the reference rPPG signal, that is, by comparing the group of pixels with the reference rPPG signal. Lock-in amplifiers are able to recover small signals from noisy data; the method leverages from the use of the lock-in amplifier to obtain a signal of high quality.

The method may further comprise, prior to obtaining the temporal rPPG signal 530, applying a filter (e.g., a spatial filter) on the groups of pixels. In other words, after estimating the reference rPPG, the method applies the filter on the groups of pixels. The method obtains the temporal rPPG signal on the filtered groups of pixels. The filter may be a spatial filter, for example a low-pass filter. The application of the low pass filter results in that quantization noise from the image is reduced, thereby improving the recognition of the rPPG signal, which is performed on individual pixels. Additionally or alternatively, the filter may be a bilateral filter, thereby improving the preservation of contours, for example a contour between the skin and the background. Thus, the method leverages from the filter to improve on the quality of the reference rPPG signal with respect to other information found on the groups of pixels. Additionally or alternatively, the filter may be an averaging filter or a Gaussian filter.

The estimation of the reference rPPG signal S20 may comprise determining a region of interest. A region of interest may be a 2D boundary (e.g., such as a bounding box, for example a rectangular bounding box) enclosing a portion of the part of the body represented on each of the sequence of images. The region of interest may enclose at least part of the skin of the body part as represented in the image. The region of interest may enclose a portion containing a full height of the part of the human body (e.g. a region from one extremity of the part of the body to other extremity of the part of the body, with respect to a predetermined direction, such as from the tip of the toe to the ankle when the part of the body is a toe). The region of interest may enclose be determined for at least one image of the sequence, optionally in all the images of the sequence.

The region of interest may be set manually (e.g., via a graphical user interface) or fully automatically, e.g., via a region of interest algorithm that sets bounding boxes on the human body part. For example, the method may set (manually or fully automatically) a dimension for a bounding box, and place it in a position enclosing the part of the body. In another example, only the dimensions of the bounding box may be set manually, whereas the positioning of the bounding box may be set automatically. The estimation may also comprise measuring a pixel mean in pixels of the region of interest. By "mean in pixels" it is meant a spatial mean of the pixels on each region of interest. Thereby, the estimation of the reference rPPG signal is a more reliable signal as the mean in pixels reduces noise between pixels of the group. The pixel mean may be obtain by applying a low pass filter to the group of pixels.

Determining the region of interest may comprise determining one or more segments of the image representing the skin from the region of interest. In other words, each segment corresponds to a respective group of pixels enclosed by the region of interest presenting a certain graphical consistency with respect to the skin of the part of the body. The method obtains the reference rPPG signal as described above, and obtain and filtering each the temporal rPPG signal as described above on the segments representing the skin. This allows the method to discard zones of the images that are not relevant to the calculation of the reference rPPG signal. Thus, the method performs less computations for obtaining the respective rPPG signal, thereby improving the efficiency of the method. For example, the method may discard portions of the image representing the background. Thus, the method is highly efficient, as the method concentrates only on portions of the image representing the skin.

The method may further comprise, prior to estimating the reference PPG signal 520, decreasing the resolution of the images to a first resolution. The first resolution is lower than the dimension of the images. In other words, the method may downsample the images from their original dimension. The method may obtain the reference rPPG signal S20 as described above, and obtain S30 and filter S40 each the temporal rPPG signal as described above. The method may further increase the resolution of the images from the first resolution to a second resolution if the respective rPPG signal satisfies a predetermined threshold. The second resolution is larger than the first resolution. The method may increase the resolution until the respective rPPG signal satisfies the predetermined threshold. Thus, the method increases the efficiency for determining the respective rPPG signal: when the respective rPPG signal is easily discernible from the groups of pixels, the method may perform the calculations fast as there are not so much pixels due to the decreased resolution. The method may thus concentrate on groups of pixels having zones where it is more difficult to discern the respective rPPG signal.

Unlike traditional manual wound assessment, the method provides particularly relevant measurements on the health of the skin as measured by the rPPG signal. The method allows a particularly fast retrieval of information and provides objective and rapid measurements for wound assessment, monitoring and even patient awareness. The output respective rPPG signal may be used with standard tools for wound assessment such as, 3D models, infrared imaging, ultrasound and hyperspectral imaging.

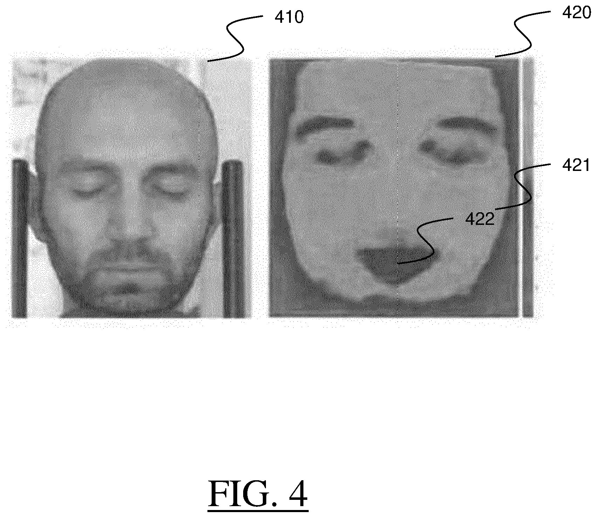

Reference is made to FIG. 4, illustrating an example of the method on a video. The method receives as input a video image 410 and outputs a 2D map 420 comprising the output indicators obtained from the respective rPPG signals.

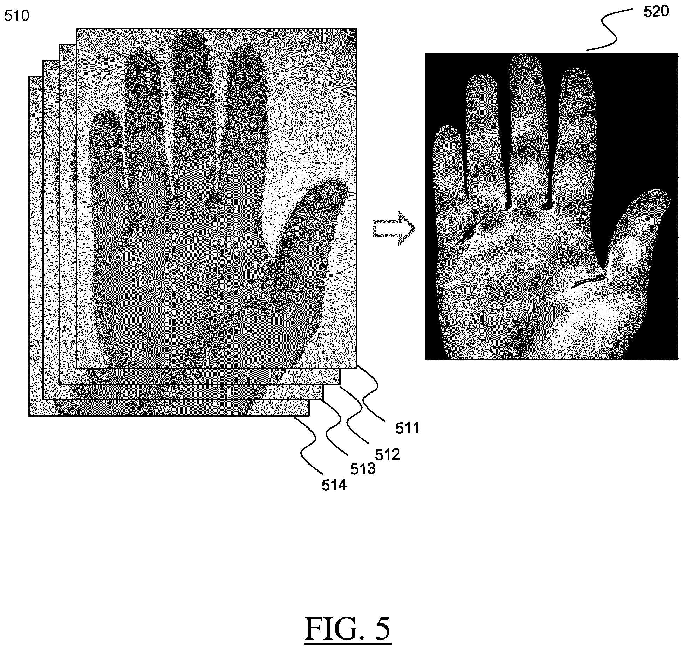

Reference is made to FIG. 5, illustrating an example of the method on a video. The method receives as input a video image 510 comprising a sequence of images 511-514. The method outputs a 2D map 520 comprising indicators of the output respective rPPG signals. The indicators represent an amplitude map of the respective rPPG signals.

## Claims

1. A computer-implemented method for monitoring one or more indicators of a part of the body from images representing the skin of the part of the body, the method comprising:
- obtaining (S10) a sequence of images representing the skin of the part of the body;
- estimating (S20) a reference remote photoplethysmography (rPPG) signal of the skin from groups of pixels of the images;
- obtaining (S30) a temporal rPPG signal for each pixel of a respective group of pixels by obtaining a linear combination of pixel characteristics of the group of pixels,
- filtering (S40) each temporal rPPG signal of the respective group of pixel based on the reference rPPG signal, thereby obtaining respective rPPG signals for each group of pixels.

2. The method of claim 1, wherein filtering (S40) each temporal rPPG signal comprises applying a lock-in amplifier on the group of pixels.

3. The method of claim 1 or 2, wherein the method further comprises, prior to obtaining the temporal rPPG signal, applying a filter on the groups of pixels, such as a low-pass filter or a bilateral filter.

4. The method of any one of claims 1 to 3, wherein the estimation of the reference rPPG signal (S20) comprises determining a region of interest enclosing the skin, and measuring a pixel mean n pixels of the region of interest.

5. The method of claim 4, further comprising determining one or more segments of the image representing the skin from the region of interest.

6. The method of any one of claims 1 to 5, wherein the sequence of images may comprise RGB images, the pixel characteristics corresponding to RGB values of a respective RGB image and/or the sequence of images may comprise monochrome images, the pixel characteristics corresponding to luminosity levels represented on a respective monochrome image.

7. The method of claim 6, wherein the sequence of images comprises RGB images, and obtaining the linear combination of pixel characteristics comprises maximizing a correlation between the RGB color values and the reference rPPG signal.

8. The method of any one of claims 1 to 7, further comprising, prior to estimating the reference PPG signal (S20), decreasing the resolution of the images to a first resolution, the first resolution being lower than the dimension of the images, obtaining (S20) the reference rPPG signal, obtaining (S30) and filtering (S40) each the temporal rPPG signal, the method further increasing the resolution of the images from the first resolution to a second resolution, the second resolution being larger than the first resolution, if the respective rPPG signal satisfies a predetermined threshold.

9. A method of use of the method of any one of claims 1 to 8, the method of use comprising:
- acquiring (S50), by an image sensor, a sequence of images representing the skin of the part of the body;
- using (S60) the method of any one of claims 1 to 6 for obtaining one or more indicators of a part of the body from images representing the skin based on the obtained respective rPPG signals.

10. The method of use of claim 9, wherein the one or more indicators comprise one or more of a perfusion index or a magnitude.

11. The method of use of claim 9 or 10, further comprising monitoring (S70) a wound on the skin based on the obtained respective rPPG signal.

12. A computer program comprising instructions which, when the program is executed by a computer, cause the computer to carry out the method of any one of claims 1 to 8 or the method of use of any one of claims 9 to 11.

13. A computer readable storage medium having recorded thereon a computer program of claim 12.

14. A system comprising a processor coupled to a memory, the memory having recorded thereon the computer program of claim 12.
